# NEW EUROPEAN PATENT SPECIFICATION

(11) **EP 1 529 844 B2**
(45) Date of publication and mention of the opposition decision: **07.11.2018**
(45) Mention of the grant of the patent: 13.08.2008
(21) Application number: 04017077.1
(22) Date of filing: 20.07.2004
(51) Int. Cl.: C12N 15/62, C12P 21/02, C07H 21/04

(54) **Complex formation for the stabilisation and purification of proteins of interest**
Komplexbildung zur Stabilisierung und Reinigung eines Proteins von Interesse
Formation d'un complexe pour la stabilisation et la purification de protéines d'interêt

(30) Priority: 21.07.2003 US 488440 P; 06.10.2003 EP 03022397
(43) Date of publication of application: 11.05.2005
(73) Proprietor: Fraunhofer-Gesellschaft zur Förderung der angewandten Forschung e.V., 80686 München (DE)
(72) Inventor: Sack, Markus, 52072 Aachen (DE); Vaquero-Martin, Carmen, 52070 Aachen (DE); Fischer, Rainer, 52156 Monschau (DE)
(74) Representative: Schiweck Weinzierl Koch Patentanwälte Partnerschaft mbB

(56) References cited:
- WO-A2-03/100021
- PUIG OSCAR ET AL: "The tandem affinity purification (TAP) method: A general procedure of protein complex purification" METHODS: A COMPANION TO METHODS IN ENZYMOLOGY, ACADEMIC PRESS INC., NEW YORK, NY, US, vol. 24, no. 3, July 2001 (2001-07), pages 218-229, XP002205606 ISSN: 1046-2023
- RIGAUT G ET AL: "A generic protein purification method for protein complex characterization and proteome exploration" NATURE BIOTECHNOLOGY, NATURE PUBLISHING, US, vol. 17, no. 10, October 1999 (1999-10), pages 1030-1032, XP002179540 ISSN: 1087-0156
- COX D M ET AL: "TANDEM AFFINITY PURIFICATION OF PROTEIN COMPLEXES FROM MAMMALIAN CELLS" BIOTECHNIQUES, EATON PUBLISHING, NATICK, US, vol. 33, no. 2, August 2002 (2002-08), pages 267-268,270, XP001154408 ISSN: 0736-6205
- HONEY S ET AL: "A novel multiple affinity purification tag and its use in identification of proteins associated with a cyclin-CDK complex." NUCLEIC ACIDS RESEARCH. 15 FEB 2001, vol. 29, no. 4, 15 February 2001 (2001-02-15), page E24, XP002320043 ISSN: 1362-4962
- FORLER DANIEL ET AL: "An efficient protein complex purification method for functional proteomics in higher eukaryotes." NATURE BIOTECHNOLOGY. UNITED STATES JAN 2003, vol. 21, no. 1, January 2003 (2003-01), pages 89-92, XP002267498 ISSN: 1087-0156
- BOULTON S J ET AL: "Use of protein-interaction maps to formulate biological questions." CURRENT OPINION IN CHEMICAL BIOLOGY. ENGLAND FEB 2001, vol. 5, no. 1, February 2001 (2001-02), pages 57-62, XP002267499 ISSN: 1367-5931
- EYCKERMAN SVEN ET AL: "Methods to map protein interactions in mammalian cells: different tools to address different questions." EUROPEAN CYTOKINE NETWORK. FRANCE 2002 JUL-SEP, vol. 13, no. 3, July 2002 (2002-07), pages 276-284, XP009024323 ISSN: 1148-5493
- STOGER, EVA ET AL: 'PRACTICAL CONSIDERATIONS FOR PHARMACEUTICAL ANTIBODY PRODUCTION IN DIFFERENT CROP SYSTEMS' MOLECULAR BREEDING vol. 9, 2002, pages 149 - 158
- ARIDGIDES, L. ET AL: 'TANDEM AFFINITY PURIFICATION OF PROTEIN COMPLEXES FROM MAMMALIAN CELLS' BIOTECHNIQUES vol. 33, 2002, pages 267 - 270
- HONEY, S. ET AL: 'A NOVEL MULTIPLE AFFINITY PURIFICATION TAG AND ITS USE IN IDENTIFICATION OF PROTEINS ASSOCIATED WITH A CYCLIN-CDK COMPLEX' NUCLEIC ACIDS RESEARCH vol. 29, no. 4, page E24, XP002320043
- BAGGA, S ET AL: 'COEXPRESSION OF MAIZE DELTA-ZEIN AND BETA-ZEIN GENES RESULTS IN STABLE ACCUMULATION OF DELTA-ZEIN IN ENDOPLASMATIC RETICULUM-DERIVED PROTEIN BODIES FORMED BY BETA-ZEIN' THE PLANT CELL no. 9, 01 September 1997, pages 1683 - 1696, XP002077186 DOI: 10.1105/TPC.9.9.1683

## Description

### BACKGROUND OF THE INVENTION

### Field of the invention

This invention relates to the production of recombinant proteins in plants. More specifically, it describes a method for altering the properties of recombinant target proteins, in short also referred to as target proteins, of interest by co-expressing a specific binding partner to the target protein, which affects the subcellular localisation of the target protein of interest. The binding partner forms a complex with its target protein, thereby linking its properties to the target protein, resulting in a target protein with altered properties and/or the emergence of novel properties in the recombinant target protein as defined in the claims.

### Background of the invention

The production of biopharmaceutical proteins in recombinant expression systems is generally cheaper, safer and more convenient than the isolation of such proteins from a natural source. Proteins expressed as recombinant molecules can also be altered by genetic engineering, which can enhance their functionality and remove undesirable properties.
Bacteria and yeast have been widely used as recombinant expression hosts, but one limitation is their inability to process proteins in the same way as animal cells. The stability and activity of recombinant animal proteins often depends on correct folding and assembly, the formation of disulphide bonds and further post-translational modifications such as acetylation, glycosylation and methylation. Bacteria carry out these processes inefficiently or not at all, and in the case of yeast the modifications are very different to those seen in animals. Bacteria also produce harmful endotoxins which may contaminate the final protein and pose a safety risk, adding considerably to the costs of downstream processing.
One method of overcoming the problems associated with microbial expression systems is to use insect or mammalian cells. While these produce more authentic proteins in terms of post-translational modification, they must be maintained as cell cultures grown in expensive bioreactors. There is also a risk that animal cells could harbour human pathogens, such as viruses or prions, or that an animal pathogen might cross the natural species barrier and become established as a human pathogen, such as has happened with BSE and the predecessors of HIV, or may contain undesirable DNA sequences, such as oncogenes. Although cheaper to maintain than mammalian cells, the same safety limitations apply to transgenic animals producing recombinant proteins in milk and other body fluids.

The search for expression systems that can produce authentic recombinant proteins but which are also convenient, economical and safe has led researchers to investigate the potential of plants, and those have been reviewed by: Fischer and Emans 2000, Transgenic Res 9, 279-299; Raskin et al. 2002, Trends Biotechnol 20, 522-531; Fischer et al. 2003, Vaccine 21, 820-825, Pogue et al. 2002, Annu. Rev. Phytopathol. 40, 45-74; Daniell et al. 2001, Trends in Plant Science 6, 219-226; Giddings et al. 2000, Nature Biotechnology 18, 1151-1155; Hood and Jilka 1999, Current Opinion in Biotechnoogy 10, 382-386; Porta and Lomonossoff 1998, Medical Virology 8, 25-41; Larrick and Thomas 2001, Biotechnology 12, 411-418. The apparatus for protein synthesis and processing in plants is very similar to that in animals, allowing complex multimeric animal proteins to be synthesised, folded, modified and assembled efficiently. Plants offer economic advantages because traditional agricultural practices and unskilled labour are sufficient for the maintenance and harvest of crops, and in many cases the infrastructure for downstream processing is already in place. Scale-up is rapid and efficient in plants, requiring only the cultivation of additional land. The costs associated with the production of a given recombinant protein in plants are therefore estimated to be 10- to 50-fold lower than in bacteria and 100- to 200-fold lower than in animal cells and transgenic animals. Plants are considered safer than the established expression systems because they do not harbour human pathogens and do not produce endotoxins that pose a threat to human health. Finally, plants offer a range of additional practical advantages including their ease of propagation, the long-term stability of recombinant proteins expressed in seeds and the potential of edible fruits, feeds and vegetables to be used for the oral administration of recombinant subunit vaccines.
Plants have been used to synthesise a wide range of biopharmaceutical proteins for diagnostic, prophylactic and therapeutic applications as well as further proteins with diverse applications in research and industry. Biopharmaceutical proteins that have been expressed in plants include human proteins, subunit vaccines and antibodies.
Human and other mammalian proteins produced in plants include growth hormone (Fischer and Emans, 2000, Transgenic Research 9, 279-299) enkephalins (Giddings et al. 2000, Nature Biotechnology 18, 1151-1155), α-interferon (Zhu et al., 1994, Virology 172, 213-222; Ohya et al. 2001, J Interferon Cytokine Res 8, 595-602), serum albumin (Giddings et al. 2000, Nature Biotechnology 18, 1151-1155; Fernandez-San Milan et al., 2003, Plant Biotech J 1, 71-79), glucocerebrosidase (Fischer and Emans, 2000, Transgenic Research 9, 279-299), granulocyte-macrophage colony stimulating factor (James et al., 2000, Protein Expr Purif19,1-139), α1-antitrypsin (Trexler et al. 2002, Biotechnol Prog 18, 501-508), epidermal growth factor (Fischer and Emans, 2000, Transgenic Research 9, 279-299) protein C (Fischer and Emans, 2000, Transgenic Research 9, 279-299) and erythropoietin (Fischer and Emans, 2000, Transgenic Research 9,279-299). Recombinant vaccines produced in plants include protein subunits of hepatitis B virus (Fischer and Emans, 2000, Transgenic Research 9, 279-299), enterotoxigenic *Escherichia coli* (Fischer and Emans, 2000, Transgenic Research 9, 279-299), Norwalk virus (Fischer and Emans, 2000, Transgenic Research 9, 279-299), measles virus (Huang et al. 2001, Vaccine 19, 2163-2171), respiratory syncytial virus (Raskin et al.;2002, Trends in Biotechnology 20, 522-531); rotavirus (Yu and Langridge 2001, Nature Biotechnol 19, 548-552), human cytomegalovirus (Fischer and Emans 2000, Transgenic Research 9, 279-299; Wright et al. 2001, Transgenic Res 10, 177-181) and cholera (Fischer and Emans 2000, Transgenic Research 9, 279-299; Yu and Langridge 2001, Nature Biotechnol 19, 548-552).
Antibody expression in plants represents a special challenge because full-size antibodies are complex, multisubunit glycoproteins. The first report describing a plant-derived antibody was that of Hiatt and colleagues (Nature 1989, 342, 76-78), which involved the production of a full-size IgG recognising phosphonate ester in transgenic tobacco. There have been many subsequent reports of antibodies expressed in plants for the purpose of immunomodulation or to confer protection against plant diseases, but the most interest has been generated by plants producing diagnostic or therapeutic antibodies. Examples include the expression of a single V_{H} domain recognising substance P in tobacco (Fischer and Emans, 2000, Transgenic Research 9, 279-299), a full-size IgG, plus Fab fragments and single chain Fv (scFv) fragments recognising creatine kinase in *Arabidopsis thaliana* and tobacco (Fischer and Emans, 2000, Transgenic Research 9, 279-299;), full size IgG and secretory IgA antibodies recognising the major adhesin of *Streptococcus mutans* in tobacco (Fischer and Emans, 2000, Transgenic Research 9, 279-299), scFv fragments recognising oxazolone in tobacco and potato (Fischer and Emans, 2000, Transgenic Research 9, 279-299), an scFv-bryodin fusion protein recognising the CD40 cell surface receptor in tobacco (Fischer and Emans, 2000, Transgenic Research 9, 279-299), a humanised IgG recognising herpes simplex virus 2 in soybean (Fischer and Emans, 2000, Transgenic Research 9, 279-299), an IgG recognising human IgG in alfalfa (Fischer and Emans, 2000, Transgenic Research 9, 279-299), a full-length IgG recognising carcinoembryonic antigen (CEA), plus various derivatives (scFv, diabody and diabody fusion with interleukin-2) in a range of plant systems (Fischer et al. 2003, Vaccine 21, 820-825), a scFv carrying the epitope of 38C13 mouse B-cell lymphoma in virus-infected tobacco (Fischer and Emans, 2000, Transgenic Research 9, 279-299), a full length IgG plus scFv and diabody derivatives recognising human chorionic gonadotropin in transgenic and transiently transformed tobacco (Fischer et al. 2003, Vaccine 21, 820-825) and a scFv fragment recognising hepatitis B virus in tobacco (Ramirez et al. 2002, FEBS Lett 469, 132-136). Several of the above plant-derived antibodies, or plantibodies, are being developed as commercial products. The most advanced is CaroRX, a secretory IgA recognising the major adhesin of the oral pathogen *S. mutans,* which can be used to prevent dental caries (Larrick et al. 2001, Current Opinions in Biotechnology 12, 411-418).
The production of antibodies and other therapeutic proteins in plants and plant-based systems has been described in the prior art. Representative examples include:
US 5,550,038 and 5,629,175, which describe constructs for the expression of physiologically active mammalian proteins in plant cells, either in tissue culture or cultivated plants. Methods for the isolation and purification of the mammalian proteins are also disclosed.
US 6,417,429, which describes the production of secretory antibodies, immunologically active derivatives or fragments thereof, within single plant cells. In one variation the immunoglobulin is a secretory IgA. The intrinsic secretion signal of the heavy chain directs the antibody into the secretory pathway. Also disclosed is a method to immunise human and animal subjects passively using the plant-derived antibodies.
US 6,303,341, which describes the coexpression of an immunoglobulin with its protection protein to yield a secretory immunoglobulin. Secretory immunoglobulin are natural hetero-multimeric proteins and are naturally secreted by epithelial cells and hepatocytes. The protection protein comprises at least amino acids 1 to 606 of the native polyimmunoglobulin receptor (pIgR). The function of the protection protein is to stabilise and protect the secreted antibody in harsh environments.
US 5,484,719 and 6,034,298 which describe the production of oral vaccines in edible plants and the administration of the oral vaccine through the consumption of the edible transgenic plants by humans and animals.
US 6,509,453, which relates to the use of oil bodies and their associated proteins as affinity matrices for the separation and purification of target molecules from samples. Target molecules include recombinant proteins expressed in plants.
While plants offer many advantages for the production of recombinant proteins, downstream processing (isolation and purification) remains a significant bottleneck that contributes most of the overall costs. The major constraints, which are important factors in any expression system, are the yields of recombinant protein produced and the quality of the end product in terms of functionality and homogeneity. Although progress has been made on these issues, the yields of recombinant protein achieved in transgenic plants are often not high enough to allow commercial exploitation. It has been established that commercial feasibility depends on recombinant protein accumulation to a level of at least 1% total soluble protein (Kusnadi et al. 1997, Biotechnol. Bioeng 56, 473-484). Therefore one of the remaining missions in the commercialisation of molecular farming in plants is the development of strategies to increase product yields to acceptable levels.
Yields of recombinant and natural proteins are influenced both by intracellular accumulation and processing losses. Intracellular accumulation depends to a large degree on the subcellular localisation of the protein. It has been shown in the case of antibodies that targeting to the endoplasmic reticulum (ER) is desirable because the molecular environment in this compartment favours protein stability (Conrad and Fiedler 1998, Plant Mol Biol 38, 101-109; Torres et al. 1999, Transgenic Res 8, 441-449). Targeting is generally achieved by the addition of a C-terminal retrieval tag, the tetrapeptide KDEL, to the recombinant protein. However, this strategy may not always be compatible with clinical applications because the antibody might be rendered immunogenic, or might display different pharmacokinetics and/or tissue distributions. For the same reason, the addition of affinity tags that would simplify purification and thus reduce processing costs (labour is a major cost factor) is undesirable for proteins intended for therapeutic use. However without such tags there are greater losses at the processing stage and costs increase.

Therefore a novel approach was invented to alter the properties of recombinant target proteins without the need of adding tags or targeting sequences to them.
The retrieval and/or targeting sequences and/or tags are instead added to an *in vivo* binding partner, which associates with the recombinant target protein. When the two proteins interact, a complex is formed and the properties of the binding partner are linked with the recombinant target protein of interest, altering the properties of the recombinant target protein.
In addition, complex formation may also result in the emergence of novel properties of the complex, the recombinant target protein and/or the binding partner. As a result, the cellular destination of the complex is affected, e.g. the complex is localised according to the cellular destination of the binding partner or the post-translational modification pattern of the target protein is altered.

### BRIEF SUMMARY OF THE INVENTION

The amount of recombinant target protein, in short the target protein, accumulating in a host cell depends on its rates of synthesis and degradation. In this invention, we provide a method for influencing the properties of a recombinant target protein by co-expressing a specific binding partner, leading to the formation of a complex between the target protein and its binding partner. A recombinant target protein is a protein normally not found in a host organism, but by providing a nucleic acid encoding said protein, the host organism is enabled to synthesise said recombinant protein.. Properties as such are the cellular targeting of the target protein, the post-translational modifications of the target protein and its amenability for phase partitioning. Complex formation is envisaged to increase the amount of the target protein primarily by decreasing the rate at which it is degraded.

The increased stability of the recombinant target protein within the complex may reflect one of several mechanisms acting alone or in combination. The recombinant target protein may become more stable because complex formation reduces the surface area that is available to the solvent, thereby protecting the protein from proteases and non-enzymatic hydrolysis. The recombinant target protein may become more stable because, while in contact with its binding partner, it is less subject to partial unfolding. For example, the instability of certain single chain Fv fragments expressed in plants and other systems is thought to reflect such partial unfolding. This can make the protein more susceptible to proteolytic attack and may also expose hydrophobic residues normally buried in the protein core, resulting in recognition by the cellular components of the protein degradation pathways. The binding partner may even be able to help the recombinant or natural target protein achieve its stable, native conformation more efficiently by interacting with correctly folded parts and preventing non-productive folding reactions. It is estimated that approximately 30% of proteins synthesised in the cell are degraded immediately because they fold incorrectly, and this proportion can be much higher for proteins that require extensive assistance from molecular chaperones.

The binding partner can also carry a genetically fused peptide tag that can be used to affect the subcellular localisation of the complex. By targeting the complex to an appropriate intracellular compartment or to an appropriate cellular structure the stability of the recombinant target protein can be further enhanced, resulting in even higher yields. For example, it has been shown that scFv fragments and other antibody derivatives accumulate to higher levels in the endoplasmic reticulum (ER) than in other compartments, such as the cytosol or apoplast. This is thought to reflect the presence of molecular chaperones in the ER, the absence of proteases and the generally more favourable chemical and physical environment. The use of the binding partner both as an stabilising entity and to provide a targeting signal could therefore increase the stability of recombinant antibodies and other recombinant target proteins in a number of ways which may act additively or synergistically.

Intracellular targeting is also required to ensure that recombinant target proteins undergo appropriate post-translational modifications. For example, full-size immunoglobulins must be targeted to the secretory pathway in order to undergo glycosylation. Due to the *in vivo* interaction between the binding partner and the target protein, sites on each molecule that are normally modified might become masked, or previously hidden sites might become accessible. In each case, this could result in a modified and/or altered and/or changed pattern and or novel pattern of biochemical modification. Such modifications that might be affected would include proteolysis, glycosylation, phosphorylation, oxidation, sulfation, hydroxylation, acylation, disulfide bridge formation and/or the attachment of non-protein prosthetic groups.

The use of the binding partner to provide a peptide tag for intracellular targeting has the advantage that it becomes unnecessary to add such a tag to the recombinant target protein itself. This is an important consideration because not all recombinant target proteins remain functional when expressed as fusions, and peptide tags may affect the clinical behaviour such as pharmacokinetics, biodistribution, and efficacy of recombinant target proteins used as pharmaceuticals, e.g. they may alter the secondary and/or tertiary structure of the protein and render it immunogenic. The binding partner can also provide a peptide tag that can be used to facilitate affinity purification of the complex, again without modifying the amino acid sequence of the target recombinant or natural protein itself. Since protein isolation and downstream processing contribute as much as 90% of the total production costs in molecular farming, efficient purification is an essential component of any production system. The complex can be purified by standard methods and the recombinant or natural target protein released from its binding partner *in vitro.* Another advantage here is that if the recombinant protein is isolated from a plant, plant tissue or cell as part of the complex, the binding partner continues to exert its protective effect against proteolytic degradation, unfolding, precipitation, oxidation and other modifications, in the extract (*in vitro*). This *in vitro* protection effect is particular useful when large volumes of extract, that require longer processing times, are processed. Suitable tags that can be used for this process include enzymes such as β-galactosidase, glutathione-S-transferase or chloramphenicol acetyltransferase; polypeptide binding proteins such as staphylococcal protein A, synthetic protein A analogue or streptococcal protein G; protein L and variants, derivatives or hybrids thereof or any other immunoglobulin binding protein, and/or the constant regions of immunoglobulins, such as the Fc part of IgG's and/or the carbohydrate-binding proteins such as the maltose-binding protein or lectins, such as concanavalin A, or carbohydrate binding proteins with a cellulose- or chitin binding domain; carbohydrate-binding proteins such as maltose-binding protein; fusion epitopes such as c-myc, RecA or FLAG; and oligo-amino acid tails such as His₆, Cys₄, Asp₅₋₁₆, Arg₅ or Phe₁₁. A single tag may be used or combinations thereof. Active fragments of any of these tags may also be used. A fragment is active, when it still has the enzymatic activity, binding activity and/or is still recognisable by antibodies directed against the full length tag.

The binding partner can also comprise a targeting sequence and/or domain leading to the attachment or recruitment of the binding partner to a non-aqueous or an aqueous phase. Non-aqueous phases in a cell are membranes such as the outer cell membrane, which is called plasmalemma in plants, vacuolar membranes, which are called tonoplast in plants, membranes of the smooth and rough ER, inner and outer membrane of the nucleus, membranes of the Golgi complex, inner and outer membrane of the organelles such as the mitochondria and chloroplast, including the thylakoid membranes; non-aqueous phases are also oilbodies, starch granules, cells walls, protein bodies, glutenin bodies, glutelin bodies and virus particles, such as plant viruses, and virus like particles. The aqueous phases within in a cell (90%) or outside a cell surround the above mentioned structures and/or may suspend, emulsify or disperse the above mentioned non-auqeous phases. Table 1 lists a selection of binding domains and/or targeting sequences that the binding partner may comprise in order to direct the complex to a particular structure listed in table 1.

With the binding partner comprising an integral transmembrane domain, membrane inserting domains or a other membrane targeting sequence, the complex is recruited to the membrane and becomes a membrane-associated or membrane-integrated protein, allowing the application of membrane-based purification strategies. Also if the binding partner comprises a polypeptide signal for attachment of GPI, palmitoylation, myristylation, farnesylation, geranylgeranylation, the complex is recruited to the membrane and becomes a membrane-associated or membrane-integrated protein, allowing the application of membrane-based purification strategies. Furthermore, if the binding partner comprises a protein domain that binds to a protein or a phospholipid located in a membrane, the complex is recruited to the membrane and becomes a membrane-associated protein, allowing the application of membrane-based purification strategies. A binding partner comprising a starch binding domain leads to the association of the complex forming between this binding partner and the recombinant target protein with cellular starch particles. In case the binding partner comprises a sequence or domain targeting it to the cell wall, such as a cellulose binding domain, the complex forming between this binding partner and the recombinant target protein associates with the cell wall. A binding partner comprising storage proteins such as a glutenin, glutelin, prolamin or fragments thereof or a polyproline helix leads to the association of the complex forming between this binding partner and the target protein with protein bodies A binding partner comprising oleosin leads to the association of the complex forming between this binding partner and the recombinant target protein with oilbodies.

**Table 1: binding domains and structures**

| **Binding domain/targeting sequence/motif** | **structure** |
|---|---|
| starch binding domain | Starch granules |
| Cellulose binding domain or fragments thereof | Cell walls |
| Glutenin or fragments thereof; glutenin binding domains or fragments thereof | Protein bodies |
| Glutelin or fragments thereof, glutelin binding domains or fragments thereof | Protein bodies |
| Prolamin or fragments thereof | Protein bodies |
| Polyproline helix | Protein bodies |
| Oleosine or fragments thereof | oilbodies |
| Membrane spanning domains / transmembrane | cellular membranes |
| - polypeptides; e.g. TCR beta chain | |
| membrane inserting domain | cellular membranes |
| - polypeptides, such as those contained in cytochrome b5, synaptobrevin, SV40 middle-T antigen | |
| Polypeptide signals for attachement of | cellular membranes |
| - GPI | |
| - palmitoylation | |
| - myristylation | |
| - farnesylation | |
| - geranylgeranylation | |
| protein domains binding to membrane components | cellular membranes |
| - proteins | |
| - phospholipids | |
| viral coat proteins or fragments thereof | virus particles and/or virus like particles |
| proteins binding to viral coat proteins or fragments thereof | virus particles and/or virus like particles |

Recruitment of the complex, and the recombinant target protein therein, to the above mentioned non-aqueous phases may also occur *in vitro,* e.g. during or after extraction, i.e. the disruption of the cells to release the complex from a different location. Phase partitioning based purification strategies that exploit the association of the complex with the non-aqueous phase may now be applied. Using appropriate conditions the first aqueous extract obtained does not contain the complex but a large number of water soluble contaminants, that can be easily removed. In a second step the complex can either be selectively released from the non-aqueous phase into a suitable aqueous phase or the complex can be disrupted; this leaves the binding partner in the non-aqueous phase and releases only the recombinant target protein into a suitable aqueous phase. Thus the different phase partitioning behaviour of the complex and the free recombinant target protein can be used to simplify and enhance significantly the downstream processing, thereby decreasing costs and increasing yields. Suitable anchor sequences that can be used for membrane integration and/or targeting include the human T cell receptor transmembrane domains (Gross and Eshar 1992, FASEB J 6, 3370-3378), glyco-phosphatidyl inositol (GPI) anchors (Gerber et al. 1992, J Biol Chem 267, 12168-12173), immunoglobulin superfamily membrane anchors, tetraspan family members (Tedder and Engel 1994, Imunol Today 15, 450-454; Wright and Tomlinson 1994, Imunol Today 15, 588-594), or membrane inserting domains from cytochrome b5, synaptobrevin, or the SV40 middle T-antigen, or any transmembrane sequence(s) from a known protein or synthesised sequences that have a similar function and can be included in the target protein by recombinant DNA technology.

The genes encoding the recombinant target protein and the binding ligand are expressed either independently from one or more nucleic acids or the genetic information for the recombinant target protein and the binding ligand are covalently linked in one gene, encoding a fusion protein comprising the recombinant target protein and the binding ligand. In the gene encoding the fusion protein the sequences for the recombinant target protein and the binding ligand are joined directly, or indirectly via a linker which may encode at least one sequence that can be used for chemical or enzymatic proteolysis. Sequences suitable for enzymatic cleavage are selected from the group of protease cleavage sites of Arg-C proteinase, Asp-N endopeptidase, caspase1-10, chymotrypsin, clostridiopeptidase B, granzyme B, enterokinase, factor Xa, glutamyl endopeptidase, LysC, papain, pepsin, proline-endopeptidase, staphylococcal peptidase I, thermolysin, thrombin, trypsin, inteins, TEV-NIa and PPV-NIa. The sequences suitable for chemical cleavage are selected from those that are attacked by BNPS-skatole ([2-(2-nitrophenylsulfenyl)-3-methylindole] such as Trp, CNBr, such as Met, formic acid, such as Asn-Pro, hydroxylamin, such as Asn-Gly, iodobenzoic acid, such as Trp and NTCB (2-nitro-5-thiocyanobenzoic acid) +Ni, such as Cys.

The fusion protein comprises at least two parts, wherein one part is the recombinant target protein and the other part is the specific binding partner. Within this fusion protein, these two parts interact, forming an intramolecular complex. The fusion protein may also form an intermolecular complex with another uncleaved or cleaved fusion protein. Alternatively, the fusion protein is proteolytically and/or chemically cleaved within the linker, releasing the recombinant target protein and the specific binding partner, and an intermolecular complex is formed between these two liberated proteins.

In an embodiment of the invention, the recombinant target protein and its binding partner form discrete intra- or intermolecular complexes. Therein the target protein and its binding partner may have any coefficient of ≥1. In a further embodiment the complexes would have the potential to form higher-order assemblies that might produce large aggregates. Conceivably, these might be deposited in special subcellular compartments similar to the protein bodies or storage vacuoles found in the endosperm tissue of cereals. The presence of large aggregates could trigger the cell to store them in separate compartments to avoid interference with other cellular processes. Since many plants produce storage proteins, protein aggregates might trigger the formation of storage compartments. In such cases, plant expression hosts that naturally possess storage proteins or specialised storage organs might be favoured for recombinant protein production. Alternatively storage could be a passive process, without any special mechanism for depositing the protein complexes. The use of membrane anchor sequences in the binding partner (see above) may promote the formation of larger aggregates in close proximity to membranes and lead to the formation of artificial cell compartments beyond cellular control.

All types of plants, both dicotyledonous and monocotyledonous, could be used for the concomitant synthesis of recombinant or natural target proteins and binding partners. Preferably it is used for solanacea, most preferably for *Nicotiana.* Proof of principle for this invention with detailed analysis was obtained by expressing two antibodies, scFv T84.66 and its diabody equivalent, along with the carcinoembryonic antigen (CEA) as the binding partner. The proteins were variously tagged with the KDEL sequence for ER-localisation, a His₆ tag for affinity purification or a transmembrane sequence from the human T-cell receptor (TCR) for recruiting the complex to a non-aqueous phase, i.e. cellular membranes. The experiments were carried out in transgenic tobacco plants and the results were confirmed using a transient expression assay in which: a) transgenic leaves expressing one of the proteins were infiltrated with *Agrobacterium* strains expressing the other; and b) non-transgenic leaves were co-infiltrated with two strains of *Agrobacterium* expressing the antibody and antigen respectively. While the transgenic plants represent a stable production system for combinations of proteins, the transient assay could be used for rapid testing of particular target protein/binding partner interactions and could be applied in a range of additional plant species, including salads, cucumbers, beans, pumpkins, methi, physalis, potato, spinach, fodder-beet and *Arabidopsis.*

The method of the present invention is particular suitable to increase the yield of a recombinant target protein which is a therapeutically active or potentially therapeutically active protein. In the sense of the present invention a therapeutically active protein is any protein that is used for the prevention or treatment of human or animal diseases. A potentially therapeutically active protein is any protein that is found to exert a certain effect during standard drug trials or during basic research and is considered to be useful for the prevention or treatment of a particular disease, but has not yet gained approval as a therapeutic for humans or animals. Therapeutically active and/or potentially therapeutically active proteins include any member of the immunoglobulin superfamily of any vertebrate species, including IgAs, IgGs, IgDs, IgEs, IgMs, such as any of said immunoglobulins directed against carcinoembryonic antigen (CEA), chimeric antibodies, humanised antibodies, and any derivative thereof such as scFvs, diabodies, bispecific scFvs, Fabs (Fab')₂ fragments, secretory components, joining chains, bi- and multivalent derivatives and bi- and multispecific derivatives and also any cytokine, interleukin, in particular human interleukin-2, collagen, hormone, serum protein, enzyme such as placental alkaline phosphatase, or cell surface protein or any derivative thereof. Furthermore the method of the present invention can also be applied to increase the yield of proteins used for diagnostic purposes and/or proteins having non-medical and non-diagnostic purposes. Proteins used for diagnostic purposes include any member of the immunoglobulin superfamily of any vertebrate species, including IgAs, IgGs, IgDs, IgEs, IgMs, such as any of said immunoglobulins directed against carcinoembryonic antigen (CEA), chimeric antibodies, humanised antibodies, and any derivative thereof such as scFvs, diabodies, bispecific scFvs, Fabs (Fab')₂ fragments, secretory components, joining chains, bi- and multivalent derivatives and bi- and multispecific derivatives thereof and also the carcinoembryonic antigen. Proteins for non-diagnostic and non-medical purposes are those used in industrial and laboratory settings, including enzymes, technical proteins, biopolymers and antibodies and derivatives thereof.

More specifically the invention provides a method for influencing the properties of a recombinant target protein in a non-human host organism. This is achieved by expressing the gene encoding the recombinant target protein in the host organism by transfecting/transforming and/or culturing/cultivating the host organism under suitable conditions and also expressing or otherwise providing a specific binding partner for the recombinant target protein in the same host, whereby the binding partner comprises a targeting sequence and/or a tag. Interactions between the recombinant target protein and the binding partner occur, yielding a complex and thereby linking the properties of the binding partner to the target protein, thus generating a target protein with novel properties and/or a complex with novel properties as defined in the claims.
The formation of the complex between the recombinant target protein with its binding partner may influence the cellular locanlisation of the recombinant target protein, the post-translational modifications of the recombinant target protein or the amenability of the recombinant target protein to phase partitioning.

In a further embodiment of the present invention, the complex formation leads to an altered pattern of post-translational modification of the recombinant target protein and/or its binding partner by changing the residues that are exposed to modifying enzymes in the cell prior to purification and/or in the *in vitro* environment following purification. In a further embodiment of the present invention, the binding partner affects the subcellular localisation of the recombinant target protein, preferably by providing a specific targeting sequence or tag directing the complex and the recombinant target protein contained therein to a specific intracellular compartment, such as the endoplasmic reticulum, or to the extracellular space, such as the apoplast.

In a further embodiment of present invention the binding partner provides a motif or domain that targets and/or integrates the complex into a cellular membrane, such as the outer cell membrane, for example the plasmalemma of plant cells, the ER or the outer and inner nuclear membranes, or recruits the complex thereto. The targeting of the complex comprising the recombinant target protein to an non-aqueous or aqueous phase makes the recombinant or natural target protein within the complex accessible to phase partitioning. The present invention allows the complex to be isolated either without disrupting the interaction between the recombinant target protein and binding partner, optionally followed by further processing, or isolation and dissociation of the complex into the recombinant or natural target protein and the binding partner, optionally followed by further processing and/or isolation of the individual proteins. In a further embodiment of the present invention the host is a microorganism or a eukaryotic species, in particular it is a plant and/or plant cell and/or plant organ or tissue and/or a plant-derived expression system. The present invention is suitable for increasing the yield of recombinant, therapeutically active or potentially therapeutically active target proteins and/or a target proteins used for diagnostic purposes and/or target proteins for non-medical purpose and/or non-diagnostic purpose. Non-medical and/or non-diagnostic purposes include laboratory methodology, industrial purposes, such as technical enzymes and/or nutritional proteins. In a further embodiment of present invention the binding partner itself is a recombinant protein. In particular, the recombinant binding partner is an antibody or antibody derivative, including IgAs, IgGs, IgDs, IgEs, IgMs, chimeric antibodies, humanised antibodies, scFvs, diabodies, bispecific scFvs, Fabs (Fab')2 fragments, secretory components, joining chains, bi- and multivalent derivatives and bi- and multispecific derivatives, preferably an antibody or derivative thereof recognising carcinoembryonic antigen (CEA); the recombinant binding partner may also be a protein assisting the folding of the recombinant target protein or a protein or domain anchored in the cellular membranes. Furthermore, the binding partner protects the recombinant target protein *in vivo* and/or *in vitro* during the purification and isolation process, and/or during prolonged storage.

### BRIEF DESCRIPTION OF THE DRAWINGS

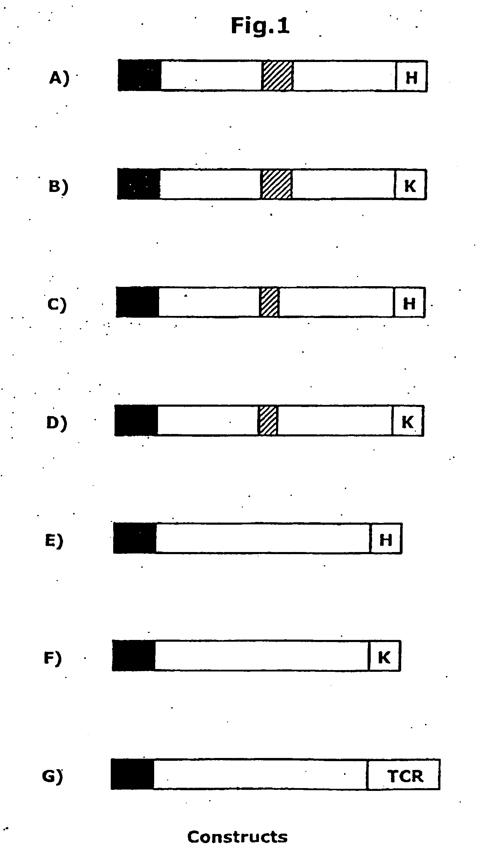

Figure 1. Schematic drawings of the constructs. A) scFv-His₆ B) scFv-KDEL C) dia-His₆ D) dia-KDEL E) CEA-His₆ F) CEA-KDEL G) CEA-TCR. All constructs are shown with the N-terminus to the left and the C-terminus to the right. Filled boxes present on all constructs represent N-terminal signal sequence that directs the protein to the secretory pathway. Hatched boxes on the antibody constructs represent the linker region between the heavy and light chain variable regions; this is shorter in the diabody constructs thus forcing the proteins to form dimers. H = His₆ tag used for affinity purification. K = KDEL tag for ER-localisation. TCR = T-cell receptor constant and transmembrane domain.

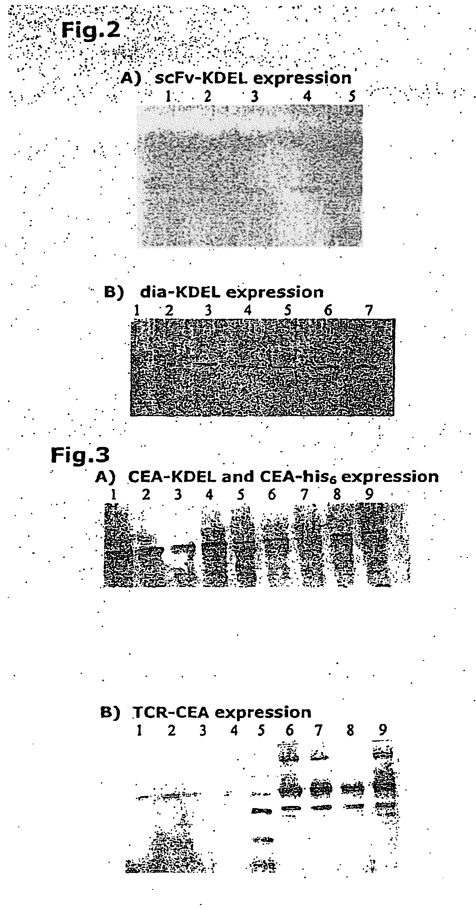

Figure 2. Western blot analysis of transgenic plants expressing A) scFv-KDEL and B) dia-KDEL, in each case detected using anti-KDEL antibodies. A) Lanes 1-4 represent parental lines expressing scFv-KDEL. Lines g (lane 2) and h (lane 4) were selected for crossing. Lane 5 shows the pre-stained markers. B) Lane 1 shows the pre-stained markers. Lanes 2-7 represent parental lines expressing dia-KDEL. Lines k (shown as duplicate samples in lanes 3 and 7) and I (lane 6) were selected for crossing.
Figure 3. Western blot analysis of transgenic plants expressing CEA constructs, in each case detected with T84.66. A) Lanes 1-4 represent parental lines expressing CEA-KDEL. Lines a (lane 2) and b (lane 4) were selected for crossing. Lanes 5-9 represent parental lines expressing CEA-His₆. Lines c and d (lanes 5 and 6) were selected for crossing. B) Parental lines expressing CEA-TCR. In lanes 1-4 proteins have been extracted without detergent and only minimal amounts of CEA-TCR can be detected. Lane 5 is the pre-stained marker. In lanes 6-9 proteins from the same four plant lines have been extracted with detergent. Lines m (lane 6) and n (lane 9) were selected for crossing.

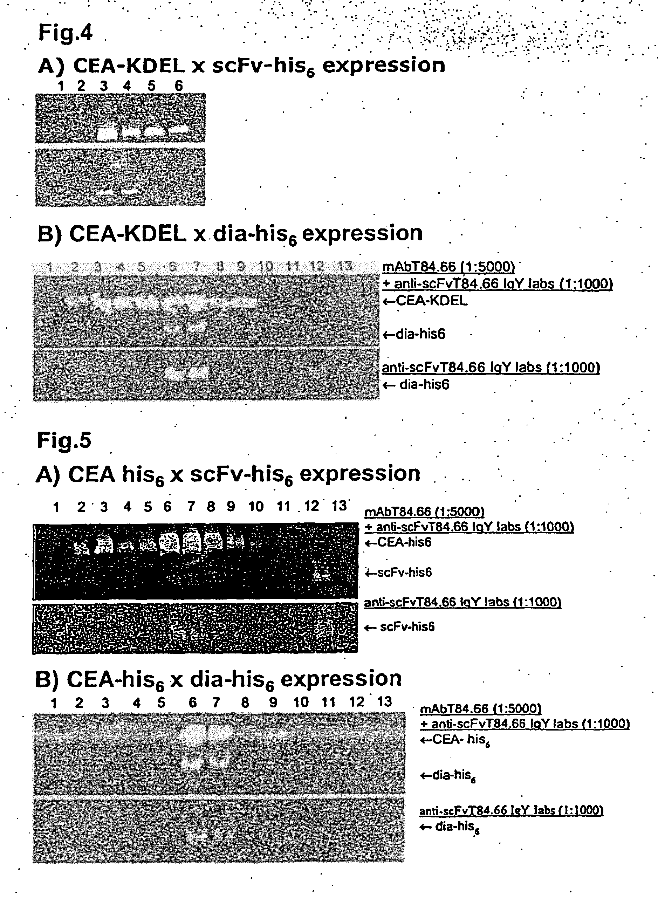

Figure 4. Western blot analysis of crude extracts from F1 plants derived from crosses between A) CEA-KDEL x scFv-His₆ and B) CEA-KDEL x dia-His₆. A) Lane 1, scFv-His₆ (f) x SR1 back cross; Lane 2, scFv-His₆ (parental; f); Lane 3, scFv-His₆ (f) x CEA-KDEL (b); Lane 4, the reverse of 3, i.e. b x f; Lane 5, CEA-KDEL (parental; b); Lane 6, CEA-KDEL (b) x SR1 back cross. Upper panel shows detection of CEA with mAbT84.66. Lower panel shows detection of scFv-His₆ with anti-His₆. B) Lane 1, marker (not visible); Lane 2, CEA-KDEL (a) x SR1; Lane 3, CEA-KDEL (a); Lane 4, CEA-KDEL (b) x SR1; Lane 5, CEA-KDEL (b); Lane 6, CEA-KDEL (a) x dia-His₆ (i); Lane 7, CEA-KDEL (b) x dia-His₆ (i); Lane 8, CEA-KDEL (a) x dia-His₆ (j); Lane 9, CEA-KDEL (b) x dia-His₆ (j); Lane 10, dia-His₆ (j); Lane 11, dia-His₆ (j) x SR1; Lane 12, dia-His₆ (i); Lane 13, dia-His₆ (i) x SR1. The upper panel shows detection of CEA-KDEL with mAbT84.66 and anti-scFvT84.66. The lower panel shows detection of dia-His₆ with anti-scFvT84.66.
Figure 5. Western blot analysis of crude extracts from F1 plants derived from crosses between A) CEA-His₆ x scFv-His₆ expression and B) CEA-His₆ x dia-His₆ expression. A) Lane 1, marker (not visible); lane 2, CEA-His₆ (c) x SR-1 (back cross); lane 3, CEA-His₆ (c); lane 4, CEA-His₆ (d) x SR-1 (back cross); lane 5, CEA-His₆ (d); lane 6, CEA-His₆ (c) x scFv-His₆ (e); lane 7, CEA-His₆ (d) x scFv-His₆ (e); lane 8, CEA-His₆ (c) x scFv-His₆ (f); lane 9, CEA-His₆ (d) x scFv-His₆ (f); 10, scFv-His₆ (e) with some spill over from lane 9 (CEA-His₆ (d)); lane 11, scFv-His₆ (e) x SR-1; lane 12, scFv-KDEL (h); lane 13, scFv-His₆ (f) x SR-1 (back cross). The upper panel shows detection of CEA-His₆ and scFv-His₆ with mAbT84.66 and anti-scFvT84.66. The lower panel shows detection of scFv-His₆ with anti-scFvT84.66. B) lane 1, marker (not visible); lane 2, CEA-His₆ (c) x SR-1 (back cross); lane 3, CEA-His₆ (c); lane 4, CEA-His₆ (d) x SR-1 (back cross); lane 5, CEA-His₆ (d); lane 6, CEA-His₆ (c) x dia-His₆ (i); lane 7, CEA-His₆ (d) x dia-His₆ (i); lane 8, CEA-His₆ (c) x dia-His₆ (j); lane 9, CEA-His₆ (d) x dia-His₆ (j), lane 10, dia-His₆ (j); lane 11, dia-His₆ (j) x SR-1 (back cross); lane 12, dia-His₆ (i); lane 13, dia-His₆ (i) x SR-1 (back cross). The upper panel shows detection of CEA-His₆ and scFv-His₆ with mAbT84.66 and anti-scFvT84.66. The lower panel shows detection of scFv-His₆ with anti-scFvT84.66.

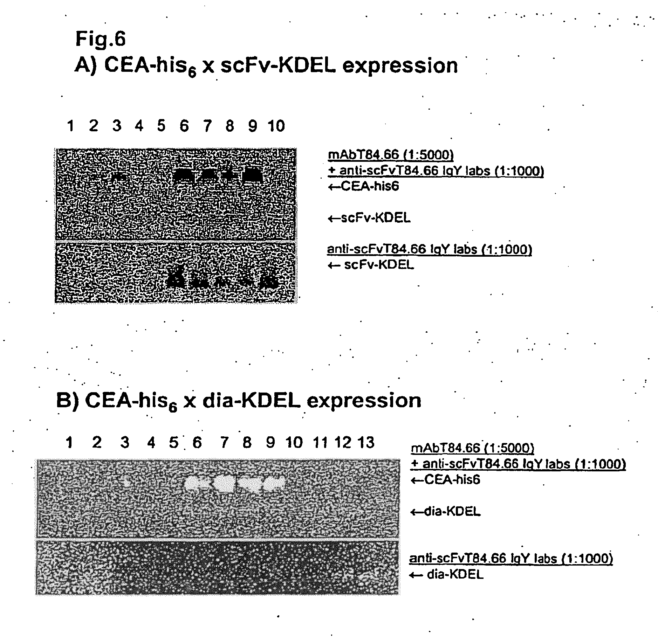

Figure 6. Western blot analysis of crude extracts from F1 plants derived from crosses between A) CEA-His₆ x scFv-KDEL and B) CEA-His₆ x dia-KDEL. A) lane 1, marker (not visible); lane 2, CEA-His₆ (c) x SR-1 (back cross); lane 3, CEA-His₆ (c); lane 4, CEA- His₆ (d) x SR-1 (back cross); lane 5, CEA-His₆ (d); lane 6, CEA-His₆ (c) x scFv-KDEL (g); lane 7, CEA-His₆ (d) x scFv-KDEL (g); lane 8, CEA-His₆ (c) x scFv-KDEL (h); lane 9, CEA-His₆ (d) x scFv-KDEL (h); lane 10, scFv-KDEL (g); lane 11, scFv- KDEL (g) x SR-1 (back cross); The upper panel shows detection of CEA- His₆ and scFv-His₆ with mAbT84.66 and anti-scFvT84.66. The lower panel shows detection of scFv-His₆ with anti-scFvT84.66. B) lane 1, marker (not visible); lane 2, CEA-His₆ (c) x SR-1 (back cross); lane 3, CEA-His₆ (c); lane 4, CEA-His₆ (d) x SR-1 (back cross); lane 5, CEA-His₆ (d); lane 6, CEA-His₆ (c) x dia-KDEL (k);lane 7, CEA-His₆ (d) x dia-KDEL (k); lane 8, CEA-His₆ (c) x dia-KDEL (I); lane 9, CEA-His₆ (d) x dia-KDEL (I); lane 10, dia-KDEL (I); lane 11, dia-KDEL (I) x SR-1 (back cross); lane 12, dia-KDEL (k); lane 13, dia-KDEL (k) x SR-1 (back cross). The upper panel shows detection of CEA-His₆ and dia-KDEL with mAbT84.66 and anti-scFvT84.66. The lower panel shows detection of dia-KDEL with anti-scFvT84.66.

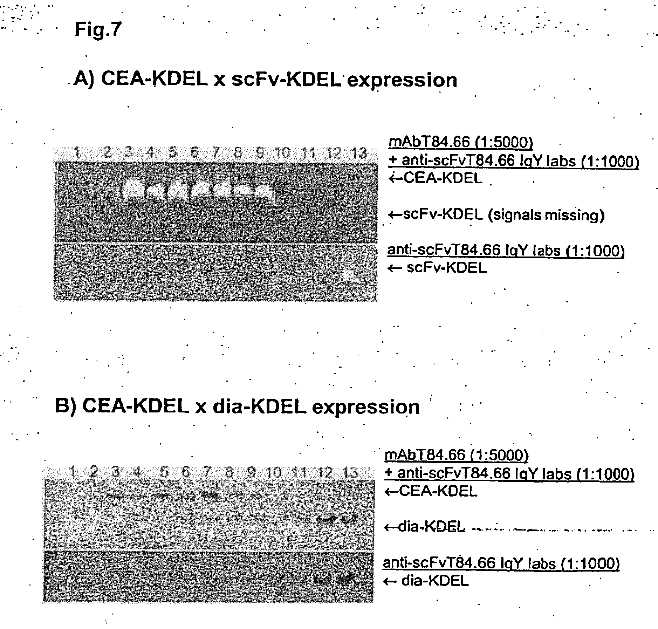

Figure 7. Western blot analysis of crude extracts from F1 plants derived from crosses between A) CEA-KDEL x scFv-KDEL and B) CEA-KDEL x dia-KDEL. A) lane 1, marker (not visible); lane 2; CEA-KDEL (a) x SR-1 (back cross); lane 3, CEA-KDEL (a); lane 4, CEA-KDEL (b) x SR-1 (back cross); lane 5, CEA-KDEL (b); lane 6, CEA-KDEL (a) x scFv-KDEL (g); lane 7, CEA-KDEL (b) x scFv-KDEL (g); lane 8, CEA-KDEL (a) x scFv-KDEL (h); lane 9, CEA-KDEL (b) x scFv-KDEL (h); lane 10, scFv-KDEL (g); lane 11, scFv-KDEL (g) x SR-1 (back cross); lane 12, scFv-KDEL (f); lane 13, scFv-KDEL (h) x SR-1 (back cross). The upper panel shows detection of CEA-KDEL and scFv-KDEL with mAbT84.66 and anti-scFvT84.66. The lower panel shows detection of scFv-KDEL with anti-scFvT84.66. B) lane 1, marker (not visible); lane 2, CEA-KDEL (a) x SR-1 (back cross); lane 3, CEA-KDEL (a); lane 4, CEA-KDEL (b) x SR-1 (back cross); lane 5, CEA-KDEL (b); lane 6, CEA-KDEL (a) x dia-KDEL (k); lane 7, CEA-KDEL (b) x dia-KDEL (k); lane 8, CEA-KDEL (a) x dia-KDEL (I); lane 9, CEA-KDEL (b) x dia-KDEL (I); lane 10, dia-KDEL (I); lane 11, dia-KDEL (I) x SR-1 (back cross); lane 12, dia-KDEL (k); lane 13, dia-KDEL (k) x SR-1 (back cross).

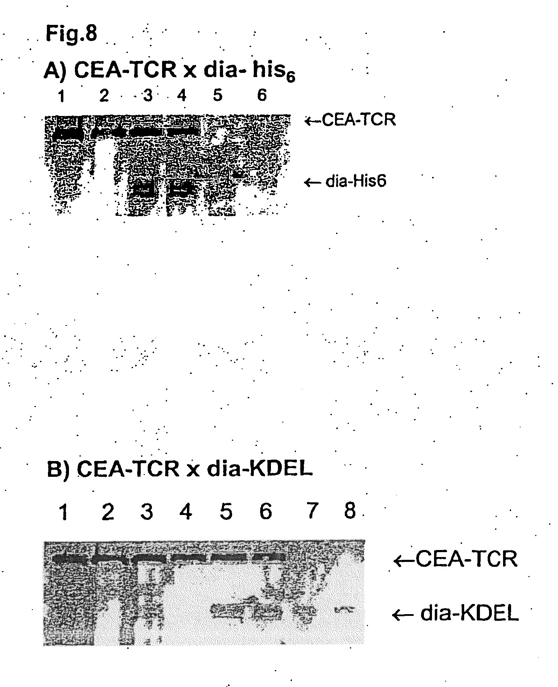

Figure 8. Western blot analysis of crude extracts from F1 plants derived from crosses between A) CEA-TCR x dia-his₆ and B) CEA-TCR x dia-KDEL. A) lane 1, CEA-TCR, parental (m); lane 2, CEA-TCR, parental (n); lane 3, CEA-TCR (m) x dia-His₆ (i) ; lane 4, CEA-TCR (n) x dia-His₆ (i); lane 5, dia-His₆, parental (i), lane 6, dia-His₆ (i) x SR1 (back cross). B) lane 1, CEA-TCR, parental (m); lane 2, CEA-TCR, parental (n); lane 3, CEA-TCR (m) x dia-KDEL (k); lane 4, CEA-TCR (n) x dia-KDEL (k); lane 5, CEA-TCR (m) x dia-KDEL (I); lane 6, CEA-TCR (n) x dia-KDEL (I); lane 7, dia-KDEL, parental (k); lane 8, dia-KDEL, parental (I). The upper panel shows detection of CEA-TCR with anti-CEA IgY and dia-His₆ with anti-scFvT84.66. The lower panel shows detection of CEA-TCR with anti-CEA IgY and dia-KDEL with anti-scFvT84.66.

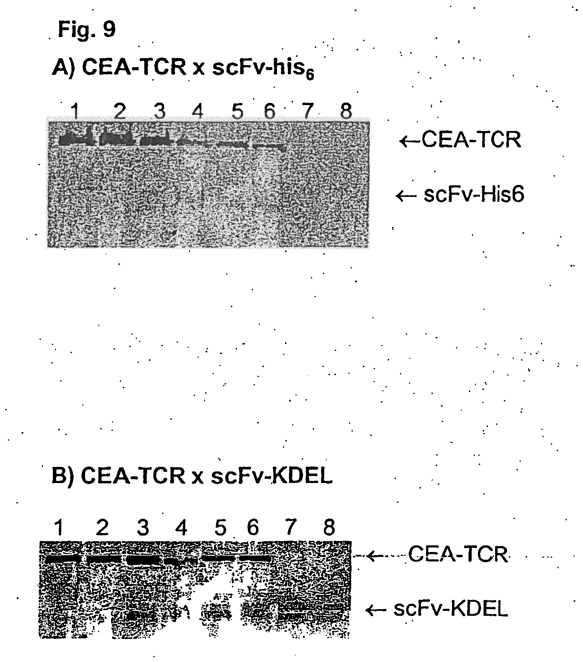

Figure 9. Western blot analysis of crude extracts from F1 plants derived from crosses between A) CEA-TCR x scFv-His₆ and B) CEA-TCR x scFv-KDEL. A) lane 1, CEA-TCR, parental (m); lane 2, CEA-TCR, parental (n); lane 3, CEA-TCR (m) x scFv-His₆ (e); lane 4, CEA-TCR (n) x scFv-His₆ (e); lane 5, CEA-TCR (m) x scFv-His₆ (f); lane 6, CEA-TCR (n) x scFv-His₆ (f); lane 7, scFv-his₆, parental (e); lane 8, scFv-his₆, parental (f). B) ) lane 1, CEA-TCR, parental (m); lane 2, CEA-TCR, parental (n); lane 3, CEA-TCR (m) x scFv-KDEL (g); lane 4, CEA-TCR (n) x scFv-KDEL (g); lane 5, CEA-TCR (m) x scFv-KDEL (h); lane 6, CEA-TCR (n) x scFv-KDEL (h); lane 7, scFv-KDEL, parental (g); lane 8, scFv-KDEL, parental (h). The upper panel shows detection of CEA-TCR with anti-CEA IgY and scFv-His₆ with anti-scFvT84.66. The lower panel shows detection of CEA-TCR with anti-CEA IgY and scFv-KDEL with anti-scFvT84.66.

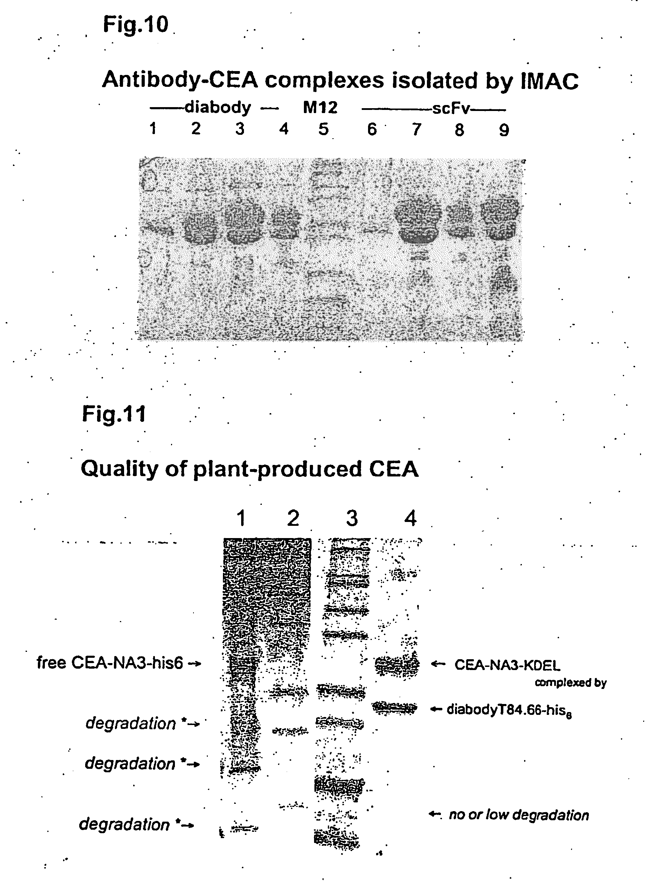

Figure 10. Sodium dodecylsulfate (SDS)-polyacrylamide gel electrophoresis (PAGE) gel of antibody-CEA complexes isolated by IMAC. Lane 1, dia-KDEL x dia-His₆ (k x i); Lane 2, CEA-KDEL x dia-His₆ (a x i); Lane 3, CEA-His₆ x dia-His₆ (c x i); Lane 4, CEA-His₆ x dia-KDEL (c x k); Lane 5, protein markers; Lane 6, scFv-KDEL x scFv-His₆ (g x e); Lane 7, CEA-KDEL x scFv- scFv-His6 (a x e); Lane 8, CEA-His₆ x scFv-His6 (c x e); Lane 9, CEA-His₆ x scFv-KDEL (c x g).

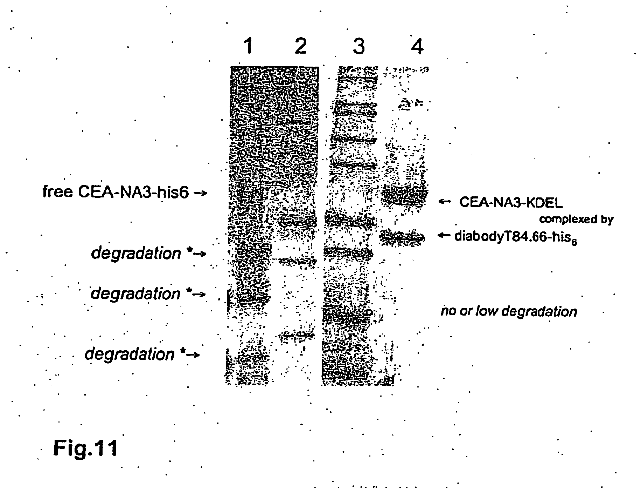

Figure 11. SDS-PAGE analysis of CEA isolated from a CEA-His₆ parental line and an F1 line (CEA-His₆ x dia-His₆). Lane 1, free CEA-His₆ from parental line; a major CEA-His₆ band and several bands representing degradation products are visible; Lane 2, protein markers; Lane 3, protein markers; Lane 4, CEA/diabody complexes isolated from F1 line (CEA-His₆ x dia-His₆); two major bands representing each component (CEA-His₆ and dia-His₆) of the complex are visible; no significant bands representing degradation products are visible.

### DETAILED DESCRIPTION OF THE INVENTION

### Constructs and parental transgenic lines

The present invention relates to a novel method for altering the properties of a natural or recombinant protein in plants by expressing a given target protein together with a specific binding partner. By sequestering the target protein into a complex, the binding partner confers enhanced stability on that protein and can be used to facilitate subcellular targeting and affinity purification. The principle was demonstrated by expressing a single chain variable fragment (scFv) antibody T84.66 (hereafter scFv) and the equivalent diabody T84.66 (hereafter dia) in transgenic plants. Both antibody derivatives recognise the carcinoembryonic antigen (hereafter CEA), which was used as the binding partner. Constructs were generated for targeting these recombinant proteins to the apoplast, to the lumen of the endoplasmic reticulum and to membranes within those compartments. The constructs are shown in schematic form in figure 1.
Parental transgenic plant lines secreting the T84.66 antibody derivatives into the apoplast were generated with constructs scFv-His₆ (figure 1 A) and dia-His₆ (figure 1 C). The scFv-His₆ antibody is a single-chain variable fragment derivative of the parental murine monoclonal antibody T84.66. The protein comprises a C-terminal tag of six consecutive histidine residues and can be purified by ion metal-chelate affinity chromatography (IMAC). An N-terminal leader peptide directs scFv-His₆ to the secretory pathway and the protein localises in the apoplast. Accumulation is rather low, and the purified scFv is unstable. It was found to degrade within a few weeks at 4°C as has been observed for other scFvs. The dia-His₆ antibody is very similar to the corresponding scFv but the linker joining the variable domains has been shortened and this forces the molecule to associate with a second one to form a stable dimer. Being a homodimer this molecule has two binding sites for the antigen. As with scFv-His₆, dia-His₆ accumulates in the apoplast and the His₆ tag fused to the C-terminus allows purification by IMAC.

Parental transgenic plant lines accumulating the antibody derivatives in the lumen of the ER were produced by expressing the recombinant genes of the constructs scFv-KDEL (figure 1 B) and dia-KDEL (figure 1 D). The ER retention signal KDEL, fused to the C-terminus of each protein, was responsible for targeting the antibodies to this compartment. The scFv-KDEL construct is similar to scFv-His₆, but has a KDEL tag instead of the His₆ tag, and so cannot be purified by IMAC. The ER-localised scFv accumulates to a level about 10 times higher than scFv-His₆. Similarly, the dia-KDEL construct has a KDEL tag instead of the His₆ tag found on the dia-His₆ construct, and also accumulates to a level approximately tenfold higher than its apoplast-secreted counterpart. Transgenic plants expressing the genes for scFv-KDEL and dia-KDEL were analysed by western blot as shown in figure 2. Transgenic plants expressing the genes for scFv-His₆ and dia-His₆ were analysed by western blot and competition ELISA (data not shown).

In a similar approach, parental transgenic lines were generated expressing the recombinant CEA gene. Transgenic plant lines secreting the CEA into the apoplast were made with the construct CEA-His₆ (figure 1 E). CEA-His₆ consists of the N- and A3-domains of CEA (CEA-NA3) fused to the C-terminal His₆ tag that allows purification by IMAC. The epitope recognised by T84.66 and its derivatives is located within the A3 domain. Due to the presence of an N-terminal leader peptide, the recombinant antigen is secreted by default into the apoplast, as confirmed by electron microscopy. Transgenic plant lines accumulating the antigen in the lumen of the ER were produced by expressing the construct CEA-KDEL (figure 1 F). This is similar to CEA-His₆ but the His₆ tag is replaced by the KDEL retrieval signal and consequently this protein cannot be purified by IMAC. EM transmission electron microscopy cannot detect CEA-KDEL in the apoplast, but ER residence has not been proven directly because immunofluorescent co-labeling in the ER is more difficult. However, indirect evidence comes from the analysis of glycan structures since the glycan chains of proteins retained in the ER do not undergo the characteristic modifications of complex glycans that take place in the Golgi apparatus.

Parental transgenic plant lines in which the CEA antigen was inserted into the cellular membranes (plasmalemma, ER and outer nuclear membrane) were created using the construct CEA-TCR (figure 1G). This construct comprises CEA-NA3 fused to the constant domains of the human T-cell receptor β-chain. This is a transmembrane protein that possesses a single membrane-spanning α-helical domain that anchors CEA in the membrane. Accumulation levels of construct CEA-TCR are between those of CEA-His₆ and CEA-KDEL (figure 3).

### Crossing

The hypothesis that an interaction between a target protein (here the antibody derivatives are the target proteins) and a binding partner (here the CEA protein) results in an increased yield of target protein was confirmed by crossing the best performing maternal lines expressing CEA with the best performing paternal lines expressing scFv or dia to produce a uniform Fl generation co-expressing the antibody and the antigen. As controls served reverse crosses and back crosses. Reverse crosses were performed to verify that the choice of motherhood and fatherhood does not influence the outcome of the crossings. The back crosses into SR1 (wildtype, non-transgenic plants) were performed to account for the gene dosage effects which potentially could lead to a two-fold reduction in the accumulation of the recombinant target protein. The crosses are summarised in table 2; therein the maternal lines are shown in the far left column and are those expressing the CEA constructs; the paternal lines are shown in the top row and are those expressing the antibody constructs; an exception are the two reverse crosses shown at the bottom of the table, wherein the maternal lines were expressing the antibody construct and the paternal lines were expressing the CEA construct.

The results of these different crosses highlight the ability of the present invention to increase the yields of a target protein, to stabilise the target protein, to target and/or direct the target protein to specific compartments or structures, to change its post-translational glycosylation pattern, allow co-purification via the tagged binding partner, and finally to allow isolation via phase partitioning. All crosses enhanced the yield of the target protein, however, the magnitude of the increase varied. In particular when IMAC purification was possible significant increases in yield (recoveries) were obtained. The ability of the present invention to increase the stability of the recombinant protein becomes obvious by comparing the results of CEA-His₆ versus CEA-His₆ x dia-His₆ in figure 11. The ability of the present invention to target a protein according to the targeting signal of its binding partner is demonstrated by all crosses, wherein one of the protein carries a KDEL tag, but not the other one. Complex formation results in the non-KDEL tagged protein also being retained in the ER via its association with its tagged binding partner. The comparison of the results of the crosses (c x i) versus (c x k) and (c x e) versus (c x g) demonstrates the ability of the present invention to induce changes in the post-translational modification pattern, in particular in the glycosylation pattern. The ability of the present invention to improve and simplify the purification of a recombinant protein by allowing its co-purification with its binding partner, was demonstrated by all crosses wherein only one of the protein carried a his tag (figure 10, table 3). The ability of the present invention to apply phase partitioning during the isolation and purification procedure was demonstrated by the crosses involving CEA-TCR constructs.

The most important crosses in terms of proving the ability of the present invention to improve accumulation and recovery of the target protein were CEA-KDEL x scFv-His₆ (a x e) and CEA-KDEL x dia-His₆ (a x i). In these crosses, the expectation was that the KDEL tag on the binding protein would retain the antibodies in the ER, resulting in a dramatic increase in antibody accumulation. As shown in figure 4 and table 3, the expected results were achieved in both cases, i.e. the levels of both scFv-His₆ and dia-His₆ were increased more than tenfold compared to the respective parental lines. The increase in protein accumulation could reflect a combination of increased stability brought about by complex formation and the altered subcellular localisation, although the latter is expected to be the predominant mechanism based on comparisons of parental lines expressing the antibodies with His₆ and KDEL tags respectively.

**Table 2: Crosses between transgenic parental lines expressing CEA and T84.66 antibody derivatives individually to produce hybrids expressing both components**

| | **ScFv-his₆ (line e,17)** | **ScFv-his₆ (line f, 36)** | **ScFv-KDEL (line g,19)** | **ScFv-KDEL (line h, 45)** | **dia-his₆ (line i, 34)** | **dia-his₆ (line j, 41)** | **dia-KDEL (line k, 11)** | **dia-KDEL (line l, 26)** | **CEA-KDEL (line a, 36)** | **CEA-KDEL (line b, 42)** |
|---|---|---|---|---|---|---|---|---|---|---|
| CEA-KDEL (line a, 36) | a:e | a:f | a:g | a:h | a:i | a:j | a:k | a:l | - | - |
| CEA-KDEL (line b, 42) | b:e | b:f | b:g | b:h | b:i | b:j | b:k | b:l | - | - |
| CEA-his₆ (line c, 3) | c:e | c:f | c:g | c:h | c:i | c:j | c:k | c:l | - | - |
| CEA-his₆ (line d, 5) | d:e | d:f | d:g | d:h | d:i | d:j | d:k | d:l | - | - |
| CEA-TCR (line m, 8) | m:e | m:f | m:g | m:h | m:i | m:j | m:k | m:l | | |
| CEA-TCR (line n, 21) | n:e | n:f | n:g | n:h | n:i | n:j | n:k | n:l | | |
| ScFv-his₆ (line e, 17) | | | | | | | | | e:a | e:b |
| scFv-his₆ (line f, 36) | | | | | | | | | f:a | f:b |

The effects of retargeting vs. complex stability can be investigated by studying F1 plants resulting from crosses of parental plants in which none of the expressed interacting proteins are retained in the ER. If stability has a significant effect on the accumulation of recombinant proteins then the CEA-His₆ x scFv-His₆ [(c x f) and (d x f)[ and CEA-His₆ x dia-His₆ [(c x i) and (d x i)] hybrids should also show higher levels of the antibody than the corresponding parental lines. Indeed there is an increase in the amount of scFv in the CEA-His₆ x scFv-His₆ [(c x e) and (d x e)] hybrid compared to the corresponding scFv-His₆ parental lines (figure 5 A). The actual yields were calculated with BCA measurements and the results are summarised in table 3. Accordingly scFv-His₆ (e; parental) alone gave a yield of less than 0.2 mg/kg. CEA-His₆ x scFv-His₆ (c x e) on the other hand gave a yield of 4.1 mg/kg. Thus the co-expression lead to an 20-fold increase in the recombinant antibody. The increase in yields are visibly even more noticable for dia (figure 5B, figure 11), suggesting either complex formation significantly stabilises this protein in particular or that there is an additional change in subcellular localisation caused by complex formation, which is not dependent on the KDEL tag. This may imply the emergence of a novel property, the alteration of the subcellular localisation without adding a specific targeting signal to the binding partner. The stability of CEA is also significantly increased in both cases. In actual terms of yields dia-His₆ (i, parental) alone gave approximately 0.4 mg/kg. When CEA-His₆ x dia-His₆ (c x i) were co-expressed, 4.9 mg/kg were obtained. That corresponds to a larger than 10-fold increase in yield.

A slight increase in the levels of both antibodies is also observed in the CEA-His₆ x scFv-KDEL [(c x g), (d x g)(c x h), and (d x h)] and CEA-His₆ x dia-KDEL [(c x k), (d x k), (d x l) and c x l)] crosses (figure 6 A, figure 6 B). Since there is no retargeting of the antibodies in this cross, the minor increase in antibody accumulation must, as above, be due to the stabilising effect of the complex. No significant increase in antibody accumulation was observed when all interacting proteins carried the KDEL sequence, i.e. CEA-KDEL x scFv-KDEL [(a x g), (b x g), (a x h) and (b x h)] and CEA-KDEL x dia-KDEL [(a x k), (b x k), (a x l) and (b x l)] (figure 7 A, figure 7 B). However, small (about two fold) increases could be masked by the lower gene dosage in the F1 plants compared to the parental line.

### Purification of expressed proteins

Although the results from western blot experiments clearly demonstrate that the co-expression of a recombinant protein and a specific binding partner, in this case scFv or dia in combination with CEA, can increase antibody accumulation levels *in planta,* it is also important to verify that the yield of antibody remains high following purification. Consequently, complexes bearing His₆ tags on one or both partners of the complex were purified from plant material by IMAC and analysed by SDS-PAGE (figure 10). Protein concentrations were determined by the BCA method and are shown in table 3.

Compared to the free His₆-tagged scFv antibody, whose concentration was too low to measure by the BCA method, all the F1 plant lines showed a significant two to ten fold increase in yield. The scFv-KDEL x scFv-His₆ cross (e x g) can be taken as a reference because no complexes are formed in this cross. A total yield of 0.66 mg per kg plant tissue was obtained from this sample (table 2, figure 10, lane 6). The scFv-His₆ x CEA-His₆ cross (c x e) results in an approximately 10-fold increase in yield to 4.1 mg, resulting from the formation of the complex but no retargeting (table 2, figure 10 lane 8). The remaining crosses, scFv-His₆ x CEA-KDEL (a x e; table 2, figure 10, lane 7) and scFv-KDEL x CEA-His₆ (c x g; table 2, figure 10, lane 9) each result in massive increases in the antibody yields, to 22.4 and 13.7 mg respectively, confirming the significant yield enhancements brought about by complex formation and the altered subcellular localisation, in combination.

**Table 3: Yields for the crosses between CEA and diabodyT84.66**

| **sample** | **concentration by BCA** (mg/ml)⁻¹ | **volume** ml⁻¹ | **total yield^{a)}** mg⁻¹ | **increase** |
|---|---|---|---|---|
| **axi** CEA-KDEL × dia-his₆ | 2.6 | 1.6 | 4.2 | 10.5 fold |
| **cxi** CEA-his₆ × dia-his₆ | 4.9 | 1 | 4.9 | 12.25 fold |
| **cxk** CEA-his₆ × dia-KDEL | 2.0 | 2.6 | 5.3 | 13.25 fold |
| **k x i** dia-KDEL × dia-his₆ | 0.55 | 1.7 | 0.94 | 2.35 fold |
| **i** (parental) dia-his₆ | 0.2 (gel estimate) | - | 0.4 | - |
| **a x e** CEA-KDEL × scFv-his₆ | 16 | 1.4 | 22.4 | 112 fold |
| **c x** CEA-his₆ × scFv-his₆ | 4.1 | 1.0 | 4.1 | 20.5 fold |
| **c x g** CEA-his₆ × scFv-KDEL | 13.7 | 1.0 | 13.7 | 68.5 fold |
| **e x g** scFv-his₆ scFv-KDEL × scFv-his₆ scFv-KDEL | 0.47 | 1.4 | 0.658 | 3.29 fold |
| **e** (parental) scFv-his₆ | n.d. | - | <0.2 | - |
| *^{a)} yields present lower estimates, recoveries were limited by the amount of Ni- NTA matrix used!For a × i specific yields of 24 mg*/*kg have been obtained from another large scale purification.* | | | | |

Similar trends were observed for the diabody although the yield increases were lower than those observed for thescFv derivative. Again the dia-KDEL x dia-His₆ cross can be taken as the base line because no antibody/antigen complexes would form in this cross. The yield in this cross was 0.94 mg of antibody per kg plant tissue (table 3, figure 10, lane 1). In the first experiment, the yield enhancement brought about by an altered subcellular localisation and complex formation (CEA-KDEL x dia-His₆) was only 2-4 fold higher than the F1 plants that did not express the CEA protein (4.2 mg) and the other crosses, in which retargeting would not be expected to occur, also showed similar enhancements (CEA-His₆ x dia-His₆, 4.9 mg; CEA-His₆ x dia-KDEL, 5.3 mg; compare figure 10, lanes 2-4). However, this was probably due to the limited amount of matrix for affinity purification because in subsequent tests it has been possible to produce yields of 24 mg per kg.

These results demonstrate three principles embodied by the invention. First, that expression as part of a complex leads to significant increases in antibody yields both through the increased stability of the complex and second, through altering the sucellular localisation; and third that the use of a binding partner containing an affinity tag can lead to efficient co-purification of the target recombinant protein. The second and third principles are particularly useful for the production of therapeutical proteins where the addition of targeting-and/or purification tags is generally not acceptable.

### Quality of the purified proteins

In another embodiment of the invention, the formation of a complex between the recombinant target protein and its specific binding partner protects both proteins from non-enzymatic degradation *in vivo* and *in vitro.* Proof of principle is demonstrated by the enhanced quality of CEA preparations from complexes compared to the protein isolated from parental lines expressing the antigen alone. Comparative SDS-PAGE analysis of CEA protein isolated from a parental line expressing CEA-His₆ and an F1 line expressing CEA- His₆ and dia-His₆ is shown in figure 11. Degradation of the free CEA protein can clearly be seen and is significantly degraded, giving rise to a number of smaller additional bands representing degradation products. In contrast, the CEA protein isolated from the hybrid line is present as a single distinct band. The other band in the same lane represents the diabody component of the complex.

### Purification of membrane complexes

When maternal CEA-TCR lines were crossed with dia-His₆, dia-KDEL, scFV-His₆ and scFv-KDEL plants, the complexes that were formed by interactions between the antibodies and their cognate antigen associated with or integrated into cellular membranes. This recruitment to a non-aequeous phase enables the use of extraction procedures and purification strategies, based on phase partitioning which has significant advantages in terms of speed, ease and costs related to downstream processing. With phase partitioning a simplified extraction procedure can be used to enrich crude preparations for the complexes.

### EXPERIMENTS

### Example 1: Cloning

Details of the procedures used to create the scFv, diabody and CEA expression constructs for transgenic plants and transient agroinfiltration assays can be found in Vaquero et al. 1999, Proc Natl Acad Sci USA 96: 11128-11133 and Schillberg et al. 2000, Molecular Breeding &, 317-326.

### Example 2: Crossing strategy

As stated above, F1 transgenic plants expressing recombinant antibodies in concert with the specific binding partner CEA were produced by crossing transgenic parental (maternal and paternal lines; see table 2) lines expressing the components of the complex individually. For most experiments, CEA constructs were expressed in the maternal lines (far left column in table 2) and antibody constructs in the paternal lines (top row in table 2). Lines selected for crossing were chosen on the basis of sufficient protein accumulation, i.e. the appropriate protein had to be detectable. A grand total of 52 crosses, as shown in table 1 were performed. All plants were grown under identical conditions and none showed any changes in phenotype or other negative effects brought about by the expression of the recombinant proteins. For each cross, five F1 plants were maintained. Leaves were collected and stored at -20°C until used for protein extraction and analysis.

### Example 3: Protein extraction for western blot analysis and purification

Initially, proteins were extracted in PBS (pH 6.0) supplemented with 5 mM 2-mercaptoethanol (2ME). Aliquots (5 µl) of each extract were loaded onto 13.5 % polyacrylamide gels for further analysis. However the extraction appeared to be incomplete and a different approach was used in further experiments. This approach featured a strongly denaturing buffer containing 9 M urea, 4.5% SDS, 7.5% 2ME and 75 mM TrisHCl (pH6.8). From these extracts, 2 µl aliquots were loaded onto polyacrylamide gels.

For western blot analysis, the gels were blotted onto nitrocellulose. Detection was mediated using either IgY-anti-scFvTS4.66 (1:1000 dilution) and horseradish peroxidase-conjugated rabbit anti-chicken antibodies (rabbit anti-chicken-HRP) (1: 5000 dilution) to detect the antibody, or mAbT84.66 (1: 2000 dilution) and goat anti-mouse-HRP (1: 5000 dilution) to detect the CEA. Alternatively, the blots were simultaneously probed with mAb anti-His₆ and mAb anti-KDEL (each at 1:2000 dilution) followed by goat anti-mouse-HRP (1: 5000 dilution). The signal was detected using a chemiluminescent substrate for HRP.

### Example 4: Purification of complexes by IMAC, quantification and quality assessment

The free dia-His₆ antibody (from parental line i) and antibody/CEA complexes in which at least one of the components carried a His₆ tag were isolated from plant extracts prepared as stated above by immobilised metal-chelate affinity chromatography (IMAC) using a resin charged with divalent nickel ions. Equal volumes of the protein extract were loaded onto a 12% SDS-polyacrylamide gel and separated under denaturing and reducing conditions to resolve the components of the complex. The gels were then stained with Coomassie brilliant blue G250 to locate the proteins bands and provide a visual estimation of relative yields and quality. The quality was assessed based on the amount of degradation as evidenced by additional bands of lower molecular weight than expected. Actual yields were determined by quantifying the proteins using the Pierce BCA microplate assay, with bovine γ-globulin as the standard. Samples were serially diluted using a starting dilution of 1:5 and concentrations were determined by plotting the slopes of the linear range.

## Claims

1. A method for altering cellular localisation, posttranslational modification or amenability to phase partitioning of a recombinant target protein, by providing a specific binding partner to the recombinant target protein which binding partner comprises a tag and/or a targeting sequence, by means of
a) expressing a gene coding for the target protein in a non-human host;
b) expressing a gene encoding the specific binding partner for the target protein or providing the specific binding partner for said target protein in a different manner in said non-human host;
c) interactions between the target protein and the binding partner yielding a complex and thereby linking the properties of the binding partner to said target protein, wherein the complex formation affects the subcellular localisation of the target protein, alters the post-translational modification pattern of the target protein and/or the binding partner, or affects phase partitioning of the target protein.

2. The method of claim 1, wherein the target protein and the specific binding partner are both part of a fusion protein.

3. The method of claim 2, wherein the fusion protein comprises at least one suitable cleavage site for enzymatic and/or chemical proteolysis.

4. The method of claims 2 to 3, wherein within the fusion protein the specific binding partner interacts with the target protein, forming an intramolecular complex.

5. The method of claims 3 to 4, wherein the fusion protein is cleaved at a proteolytic cleavage site, releasing the recombinant target protein and the binding partner.

6. The method of claims 3 to 5, wherein the binding partner of one fusion protein interacts with the target protein part of another fusion protein or with a liberated target protein, forming an intermolecular complex.

7. The method of claim 5, wherein the released target protein and the binding partner form an intermolecular complex.

8. The method of claim 1, wherein the target protein and the binding partner are not fused to each and are encoded by separate genes.

9. The method of claim 1, wherein the altered pattern of post-translational modification concerns proteolysis, glycosylation, phosphorylation, oxidation, sulfation, hydroxylation, acylation, disulfide bridge formation and/or the attachment of non-protein prosthetic groups.

10. The method of claims 1 to 9, wherein the binding partner comprises a targeting sequence, and/or a tag leading to the attachment or recruitment of the complex to a non-aqueous or an aqueous phase.

11. The method of claim 10, wherein the complex with the target protein contained therein is directed to an extracellular space, such as the apoplast, the medium supernatant of suspension and/or hairy root cultures, an intracellular compartment such as the ER, golgi, nucleus, vacuole, glyoxisomes, lysosomes and/or the cell wall, starch granules, protein bodies and/or oil bodies or to an intra- or extracellular structure, such as the cellular membranes including the outer cell membrane, vacuolar membranes, membranes of the smooth and rough ER, inner and outer membrane of the nucleus, membranes of the Golgi complex and/or inner and outer membranes of organelles such as the mitochondria and the chloroplasts, including the thylakoid membranes, organelles such as mitochondria.

12. The method of claims 1 to 11, wherein the binding partner provides a motif or domain that targets and/or integrates the complex into a cellular membrane, such as those listed in claim 11, or recruits the complex thereto.

13. The method of claims 1 to 12, wherein the host is a microorganism or a eukaryotic species.

14. The method according to claim 13, wherein the host is a plant and/or plant cell and/or plant organ or tissue and/or a plant-derived expression system.

15. The method of claims 13 to 14, wherein the host is infected by a virus or by agrobacterium tumefaciens, to express either the recombinant target protein and/or the binding partner.

16. The method of claims 1 to 15, wherein the target protein is a therapeutically active or potentially therapeutically active protein and/or a protein used for diagnostic purposes and/or a protein for non-medical use.

17. The method of claims 1 to 16, wherein the binding partner is a recombinant protein.

## Patentansprüche

1. Verfahren zur Veränderung zellulärer Lokalisierung, posttranslationaler Modifikation oder Fähigkeit der Auftrennung zwischen Phasen eines rekombinanten Zielproteins durch Bereitstellen eines spezifischen Bindungspartners für das rekombinante Zielprotein, wobei der Bindungspartner eine Markierung und/oder eine Zielsteuerungssequenz umfasst, mittels
a) Exprimierens eines Gens, das für das Zielprotein codiert, in einem nichthumanen Wirt;
b) Exprimierens eines Gens, das den spezifischen Bindungspartner für das Zielprotein codiert, oder Bereitstellen des spezifischen Bindungspartners für das Zielprotein auf andere Weise in dem nichthumanen Wirt;
c) Wechselwirkungen zwischen dem Zielprotein und dem Bindungspartner, was einen Komplex ergibt, und dadurch Verknüpfen der Eigenschaften des Bindungspartners mit dem Zielprotein, wobei die Komplexbildung die subzelluläre Lokalisierung des Zielproteins beeinflusst, das posttranslationale Modifikationsmuster des Zielproteins und/oder des Bindungspartners verändert, oder die Auftrennung des Zielproteins zwischen Phasen beeinflusst.

2. Verfahren nach Anspruch 1, wobei das Zielprotein und der spezifischen Bindungspartner beide Bestandteil eines Fusionsproteins sind.

3. Verfahren nach Anspruch 2, wobei das Fusionsprotein wenigstens eine geeignete Spaltungsstelle für die enzymatische und/oder chemische Proteolyse umfasst.

4. Verfahren nach Anspruch 2 bis 3, wobei innerhalb des Fusionsproteins der spezifische Bindungspartner mit dem Zielprotein interagiert, wobei ein intramolekularer Komplex gebildet wird.

5. Verfahren nach Anspruch 3 bis 4, wobei das Fusionsprotein an einer proteolytischen Spaltungsstelle gespalten wird und dadurch das rekombinante Zielprotein und der Bindungspartner freigesetzt werden.

6. Verfahren nach Anspruch 3 bis 5, wobei der Bindungspartner eines Fusionsproteins mit dem Zielproteinteil eines anderen Fusionsproteins oder mit einem freigesetzten Zielprotein interagiert, wobei ein intermolekularer Komplex gebildet wird.

7. Verfahren nach Anspruch 5, wobei das freigesetzte Zielprotein und der Bindungspartner einen intermolekularen Komplex bilden.

8. Verfahren nach Anspruch 1, wobei das Zielprotein und der Bindungspartner nicht miteinander fusioniert sind und durch getrennte Gene codiert werden.

9. Verfahren nach Anspruch 1, wobei das geänderte Muster der posttranslationalen Modifikation die Proteolyse, Glycosylierung, Phosphorylierung, Oxidation, Sulfatierung, Hydroxylierung, Acylierung, Disulfidbrückenbildung und/oder die Bindung von prosthetischen Nichtproteingruppen betrifft.

10. Verfahren nach Anspruch 1 bis 9, wobei der Bindungspartner eine Zielsteuerungssequenz und/oder eine Markierung umfasst, die zu der Bindung oder Rekrutierung des Komplexes an eine bzw. zu einer nichtwässrige(n) oder wässrige(n) Phase führt.

11. Verfahren nach Anspruch 10, wobei der Komplex mit dem darin enthaltenen Zielprotein in einen extrazellulären Raum, wie den Apoplasten, das überstehende Medium von Suspensions- und/oder Hairy-Root-Kulturen, ein intrazelluläres Kompartiment wie das ER, den Golgi-Apparat, Zellkern, eine Vakuole, Glyoxysomen, Lyosomen und/oder die Zellwand, Stärkekörnchen, Proteinkörperchen, und/oder Ölkörperchen oder in eine intra- oder extrazelluläre Struktur, wie die Zellmembranen, einschließlich der äußeren Zellmembran, Vakuolenmembranen, Membranen des glatten und rauen ER, der inneren und äußeren Membran des Zellkerns, der Membranen des Golgi-Apparats und/oder der inneren und äußeren Membranen von Organellen, wie den Mitochondrien und den Chloroplasten, einschließlich der Thyklakoidmembranen, Organellen, wie Mitochondrien, gelenkt wird.

12. Verfahren nach Anspruch 1 bis 11, wobei der Bindungspartner ein Motiv oder eine Domäne liefert, das bzw. die den Komplex in eine Zellmembran, wie die in Anspruch 11 aufgeführten, lenkt und/oder integriert, oder den Komplex dorthin rekrutiert.

13. Verfahren nach Anspruch 1 bis 12, wobei der Wirt ein Mikroorganismus oder eine eukaryontische Spezies ist.

14. Verfahren nach Anspruch 13, wobei der Wirt eine Pflanze und/oder Pflanzenzelle und/oder ein Pflanzenorgan oder -gewebe und/oder ein von Pflanzen abgeleitetes Expressionssystem ist.

15. Verfahren nach Anspruch 13 bis 14, wobei der Wirt von einem Virus oder von *Agrobacterium tumefaciens* infiziert ist, so dass er entweder das rekombinante Zielprotein und/oder den Bindungspartner exprimiert.

16. Verfahren nach Anspruch 1 bis 15, wobei das Zielprotein ein therapeutisch wirksames oder potentiell therapeutisch wirksames Protein und/oder ein für diagnostische Zwecke verwendetes Protein und/oder ein Protein für nichtmedizinische Verwendung ist.

17. Verfahren nach Anspruch 1 bis 16, wobei der Bindungspartner ein rekombinantes Protein ist.

## Revendications

1. Procédé pour modifier la localisation cellulaire, la modification post-traductionnelle ou la possibilité de la séparation entre phases d'une protéine cible recombinée en fournissant un partenaire de liaison spécifique à la protéine cible recombinée comprenant un marqueur et/ou une séquence cible, au moyen
a) de l'expression d'un gène codant pour la protéine cible chez un hôte non humain;
b) de l'expression d'un gène codant le partenaire de liaison spécifique pour la protéine cible ou la mise à disposition du partenaire de liaison spécifique pour ladite protéine cible d'une manière différente chez ledit hôte non humain;
c) d'interactions entre la protéine cible et le partenaire de liaison produisant un complexe et associant ainsi les propriétés du partenaire de liaison à ladite protéine cible, la formation du complexe affectant la localisation subcellulaire de la protéine cible, altérant le profil de modification post-traductionnelle de la protéine cible et/ou le partenaire de liaison, ou affectant la séparation entre phases de la protéine cible.

2. Procédé selon la revendication 1, dans lequel la protéine cible et le partenaire de liaison spécifique font tous deux partie d'une protéine de fusion.

3. Procédé selon la revendication 2, dans lequel la protéine de fusion comprend au moins un site de clivage approprié pour une protéolyse enzymatique et/ou chimique.

4. Procédé selon les revendications 2 à 3, dans lequel, dans la protéine de fusion, le partenaire de liaison spécifique interagit avec la protéine cible, formant un complexe intramoléculaire.

5. Procédé selon les revendications 3 à 4, dans lequel la protéine de fusion est clivée au niveau d'un site de clivage protéolique, libérant la protéine cible recombinée et le partenaire de liaison.

6. Procédé selon les revendications 3 à 5, dans lequel le partenaire de liaison d'une protéine de fusion interagit avec la partie de protéine cible d'une autre protéine de fusion ou avec une protéine cible libérée, formant un complexe intermoléculaire.

7. Procédé selon la revendication 5, dans lequel la protéine cible libérée et le partenaire de liaison forment un complexe intermoléculaire.

8. Procédé selon la revendication 1, dans lequel la protéine cible et le partenaire de liaison ne sont pas fusionnés ensemble et sont codés par des gènes séparés.

9. Procédé selon la revendication 1, dans lequel le profil modifié de modification post-traductionnelle concerne la protéolyse, la glycosylation, la phosphorylation, l'oxydation, la sulfatation, l'hydroxylation, l'acylation, la formation de ponts disulfures et/ou la fixation de groupes prosthétiques non protéiques.

10. Procédé selon les revendications 1 à 9, dans lequel le partenaire de liaison comprend une séquence cible et/ou un marqueur conduisant à la fixation ou au recrutement du complexe dans une phase non aqueuse ou aqueuse.

11. Procédé selon la revendication 10, dans lequel le complexe avec la protéine cible contenue dans celui-ci est dirigé vers un espace extracellulaire, tel que l'apoplasme, le surnageant du milieu des cultures de la suspension et/ou de chevelu racinaire, un compartiment intracellulaire tel que le réticulum endoplasmique (RE), l'appareil de Golgi, le noyau, la vacuole, les glyoxysomes, les lysosomes et/ou la paroi cellulaire, les grains d'amidon, les corps protéiques et/ou les corps lipidiques, ou vers une structure intra- ou extra-cellulaire telle que les membranes cellulaires comprenant la membrane cellulaire externe, les membranes vacuolaires, les membranes du RE lisse et du RE rugueux, les membranes internes et externes du noyau, les membranes de l'appareil de Golgi, et/ou les membranes internes et externes d'organites tels que les mitochondries et les chloroplastes, comprenant les membranes thylakoïdes, les organites tels que les mitochondries.

12. Procédé selon les revendications 1 à 11, dans lequel le partenaire de liaison fournit un motif ou un domaine qui cible et/ou intègre le complexe dans une membrane cellulaire telle que celles énumérées à la revendication 11, ou recrute le complexe à celle-ci.

13. Procédé selon les revendications 1 à 12, dans lequel l'hôte est un microorganisme ou une espèce eucaryote.

14. Procédé selon la revendication 13, dans lequel l'hôte est une plante et/ou une cellule végétale et/ou un organe ou un tissu végétal et/ou un système d'expression d'origine végétale.

15. Procédé selon les revendications 13 à 14, dans lequel l'hôte est infecté par un virus ou par *Agrobacterium tumefaciens* pour exprimer la protéine cible recombinée et/ou le partenaire de liaison.

16. Procédé selon les revendications 1 à 15, dans lequel la protéine cible est une protéine active sur le plan thérapeutique ou potentiellement active sur le plan thérapeutique et/ou une protéine utilisée à des fins diagnostiques et/ou une protéine destinée à une utilisation non médicale.

17. Procédé selon les revendications 1 à 16, dans lequel le partenaire de liaison est une protéine recombinée.
